(19) Europäisches Patentamt
European Patent Office
Office européen des brevets

(11) Veröffentlichungsnummer : **0 550 907 B1**

# (12) EUROPÄISCHE PATENTSCHRIFT

(45) Veröffentlichungstag der Patentschrift : **18.01.95 Patentblatt 95/03**

(51) Int. Cl.$^6$ : **G03C 7/392**

(21) Anmeldenummer : **92122081.0**

(22) Anmeldetag : **28.12.92**

## (54) Fotografisches Aufzeichnungsmaterial.

(30) Priorität : **09.01.92 DE 4200322**

(43) Veröffentlichungstag der Anmeldung : **14.07.93 Patentblatt 93/28**

(45) Bekanntmachung des Hinweises auf die Patenterteilung : **18.01.95 Patentblatt 95/03**

(84) Benannte Vertragsstaaten : **BE DE FR GB IT**

(56) Entgegenhaltungen :
**EP-A- 0 294 149**
**DE-A- 2 042 533**

(73) Patentinhaber : **Agfa-Gevaert AG**
**Kaiser-Wilhelm-Allee**
**D-51373 Leverkusen (DE)**

(72) Erfinder : **Odenwälder, Heinrich, Dr.**
**Am Arenzberg 37**
**W-5090 Leverkusen 3 (DE)**
Erfinder : **Öhlschläger, Hans, Dr.**
**Am Katterbach 34**
**W-5060 Bergisch Gladbach 2 (DE)**
Erfinder : **Stetzer, Thomas, Dr.**
**Seidenweberstrasse 50**
**W-4018 Langenfeld (DE)**

EP 0 550 907 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein fotografisches Aufzeichnungsmaterial auf einem transparenten Träger, das sich durch hohe Interimage-Effekte sowie durch verbesserte Schärfe und Körnigkeit auszeichnet.

Es ist bekannt, daß alle modernen farbfotografischen Aufzeichnungsmaterialien nach dem Prinzip der subtraktiven Farberzeugung arbeiten. Das allgemeine Prinzip dieser fotografischen Farbaufzeichnung besteht darin, im Positivbild gelbe, purpurne und blaugrüne Farbstoffe so zu erzeugen, daß ihre Konzentrationen im umgekehrten Verhältnis zur Intensität des eingestrahlten blauen, grünen und roten Lichtes stehen.

Die Farbstoffbildung beruht darauf, daß an den belichteten Stellen während der Entwicklung infolge der Reduktion von Silberhalogenid zu Silber Entwickleroxidationsprodukte gebildet werden, die sich mit farblosen Komponenten, den sogenannten Farbkupplern, in den Schichten zu Bildfarbstoffen umsetzen. Je nach der Natur dieser Farbkuppler erhält man aus β-Ketoaniliden gelbe, aus Pyrazolonen purpurne und aus α-Naphtholen beziehungsweise Phenolen blaugrüne Bildfarbstoffe.

Die Art der Substitution und Einbringung der Farbkuppler in die fotografische Schicht beeinflußt entscheidend die Absorption der Bildfarbstoffe und damit die Farbwiedergabe. Im Idealfall sollten die drei Bildfarbstoffe jeweils nur in einem Spektraldrittel absorbieren. Das ist in der Praxis allerdings nicht zu realisieren. Besonders ungünstig wirken sich Überlappungen der Absorptionsbereiche und Nebenabsorptionen in anderen Spektralbereichen aus, die zu Farbverfälschungen führen.

Zur Überwindung derartiger Fehler wurden in der Vergangenheit dem fotografischen Aufzeichnungsmaterial schon Verbindungen zugesetzt, die einen sogenannten Interimage-Effekt bewirken sollen (z.B. US-A-3 536 487).

Der Interimage-Effekt ist unter anderem näher beschrieben in Hanson et al., Journal of the Optical Society of America, Bd. 42, S. 663 bis 669 und in A. Thiels, Zeitschrift für Wissenschaftliche Photographie, Photophysik und Photochemie, Bd. 47, S. 106 bis 118 und S. 246 bis 255.

Es ist ebenfalls bekannt, daß Interimage-Effekte durch den Einbau von DIR-Verbindungen (DIR = development inhibitor releasing) in die Schichten eines fotografischen Materials erzielt werden können. Bei den DIR-Verbindungen kann es sich um solche handeln, die unter Abspaltung eines Inhibitorrestes mit dem Oxidationsprodukt eines Farbentwicklers zu einem Farbstoff reagieren (DIR-Kuppler), oder um solche, die den Inhibitor freisetzen ohne gleichzeitig einen Farbstoff zu bilden. Letztere werden auch als DIR-Verbindungen im engeren Sinne bezeichnet. Bei freigesetzten Entwicklungsinhibitoren handelt es sich in der Regel um heterocyclische Mercaptoverbindungen oder um Derivate des Benzotriazols.

Auch bekannt ist aus DE-A-1 447 569 (= US-A-3 362 826) der Zusatz bestimmter heterocyclischer Mercaptoverbindungen zur Barytageschicht eines schwarzweißfotografischen Papiers. Diese ein- und zweikernigen Mercaptooxadiazole sollen die Entstehung eines Gelbschleiers nach der Verarbeitung des Materials verhindern.

In Farbumkehrmaterialien sind DIR-Verbindungen jedoch nur von geringem Wert zur Steuerung von Interimage- und Kanteneffekten, da sie erst im Farbentwickler aktiv sind und Inhibierungseffekte in der Farbentwicklung der Umkehrverarbeitung nur sehr schwer zu errreichen sind.

Im Erstentwickler der Farbumkehrentwicklung, einem Schwarzweiß-Entwickler, wirksam sind aber die sogenannten IRD-Verbindungen (IRD = inhibitor releasing developer), z.B. die DIR-Hydrochinone aus DE-A-2 952 280. Durch ihre Inhibierungseffekte lassen sich hohe Interimage- und Kanteneffekte erzielen. Bis heute konnte aber ein entscheidender Nachteil dieser Verbindungen, nämlich ihre Instabilität, noch nicht beseitigt werden, Die Instabilität bewirkt bei der Lagerung des fotografischen Materials, besonders unter heißen und feuchten Bedingungen, gen, eine Veränderung der Eigenschaften des fotografischen Materials, z.B. kann es zu einem Verlust an Empfindlichkeit kommen.

DE-A-2 042 533 beschreibt ein fotografisches Material, dessen Stabilität durch Zusatz von heterocyclischen Thiokohlensäureestern oder Urethanen verbessert wird. In EP-A-294 149 wird der Einsatz von heterocyclischen Mercaptoverbindungen, z.B. Mercaptooxadiazolen, in fotografischen Materialien beschrieben, wobei eine hohe Empfindlichkeit und ein niedriger Schleier beobachtet wird.

Der Erfindung liegt die Aufgabe zugrunde, ein fotografisches Aufzeichnungsmaterial bereitzustellen, das eine einen Interimage-Effekt bewirkende Verbindung enthält, aber die bisher damit verbundenen Nachteile nicht aufweist.

Gegenstand der Erfindung ist daher ein fotografisches Aufzeichnungsmaterial enthaltend einen transparenten Träger mit mindestens einer lichtempfindlichen Silberhalogenidemulsionsschicht sowie gegebenenfalls üblichen Zwischen- und Schutzschichten, dadurch gekennzeichnet, daß das fotografische Aufzeichnungsmaterial in mindestens einer der genannten Schichten eine Verbindung der Formel I

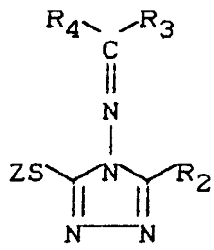

(I)

enthält, worin

- $R_2$ Wasserstoff, einen unsubstituierten oder substituierten Alkylrest, einen unsubstituierten oder substituierten Arylrest, einen unsubstituierten oder substituierten Alkylthiorest,
- $R_3$ Wasserstoff, einen unsubstituierten oder substituierten Alkylrest,
- $R_4$ einen unsubstituierten oder substituierten Alkylrest, einen unsubstituierten oder substituierten Arylrest, einen unsubstituierten oder substituierten heterocyclischen Rest,
- Z Wasserstoff, ein Kation oder eine Vorläuferverbindung davon, die bei der Entwicklung nicht-bildmäßig die Mercaptoverbindung freisetzen kann,

bedeuten.

Wenn es sich bei einem der Reste $R_2$ bis $R_4$ um einen Alkylrest handelt, ist dies vorzugsweise ein Alkylrest mit 1 bis 6 Kohlenstoffatomen. Die Alkylreste können geradkettig, verzweigt oder cyclisch sein.

Bei einem Arylrest handelt es sich vorzugsweise um einen Phenylrest. Bei einem Aralkylrest handelt es sich vorzugsweise um einen Benzylrest.

Im Fall eines heterocyclischen Restes sind Reste bevorzugt, die sich von Furan, Thiophen, Pyridin oder Thiazol ableiten.

Bei einem Substituenten handelt es sich vorzugsweise um Sauerstoff, Schwefel, Stickstoff, Fluor, Chlor oder Brom. Besonders bevorzugte Reste sind z.B. Hydroxy, Mercapto, Alkoxy, Alkylthio, Alkoxycarbonyl oder Amino.

Wenn es sich bei Z um ein Kation handelt, ist dies vorzugsweise ein Natrium-, Magnesium-, Calcium- oder Ammoniumion. Im Fall einer Vorläuferverbindung ist Z eine Gruppe, die bei der Entwicklung bei pH 8-14 abgespalten und durch H ersetzt wird, wie z.B. ein Acetyl-, Chloracetyl-, Methylsulfonylethyl- oder Cyanoethylrest.

Besonders vorteilhafte Mercaptotriazole der Formel I sind in Tabelle 1 angegeben.

Tabelle 1

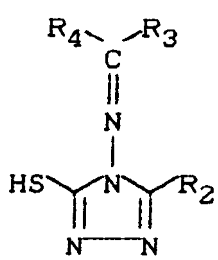

| $R_2$ | $R_3$ | $R_4$ | Verbindung |
|---|---|---|---|
| H | H | 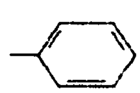 | B-1 |

| $R_2$ | $R_3$ | $R_4$ | Verbindung |
|---|---|---|---|
| H | H | 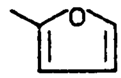 | B-2 |
| H | H | 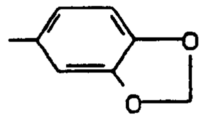 | B-3 |
| H | H | 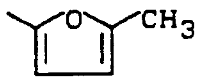 | B-4 |
| $C_2H_5$ | H | 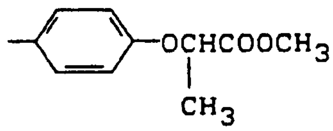 | B-5 |
| $CH_3$ | H | 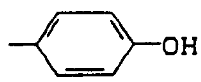 | B-6 |
| H | H | 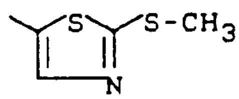 | B-7 |
| $-SCH_3$ | H | 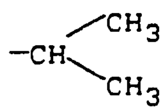 | B-8 |
| $-S-CH_2-COOC_2H_5$ | H | 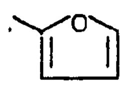 | B-9 |
| 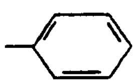 | $CH_3$ | $CH_3$ | B-10 |
| H | H | 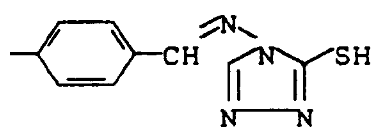 | B-11 |

Bei einem farbfotografischen Material werden mit den erfindungsgemäßen Verbindungen ausgezeichnete Interimage- und Kanteneffekte erzielt, die zu hervorragender Farbwiedergabe, Detailwiedergabe und Körnigkeit führen. Bei einem Schwarzweiß-Material werden mit den erfindungsgemäßen Verbindungen eine hohe Schärfe und eine ausgezeichnete Körnigkeit erhalten.

Obwohl die erfindungsgemäßen Verbindungen jeder beliebigen Schicht des fotografischen Materials zugesetzt werden können, werden die Verbindungen in einer bevorzugten Ausführungsform einer lichtempfindlichen Silberhalogenidemulsionsschicht zugegeben.

In einer weiteren bevorzugten Ausführungsform handelt es sich um ein farbfotografisches Aufzeichnungsmaterial mit mindestens einer rotempfindlichen, mindestens einer grünempfindlichen und mindestens einer

blauempfindlichen Silberhalogenidemulsionsschicht, die in der angegebenen Reihenfolge mindestens einen Blaugrünkuppler, mindestens einen Purpurkuppler und mindestens einen Gelbkuppler enthalten.

In einer besonders bevorzugten Ausführungsform handelt es sich bei dem erfindungsgemäßen Material um ein Colorumkehrmaterial auf einem transparenten Träger.

Als Silberhalogenide der farbkupplerhaltigen und der farbkupplerfreien Silberhalogenidemulsionsschichten kommen AgBr, AgBrI, AgBrCl, AgBrClI und AgCl in Betracht.

Die Silberhalogenidemulsionen können negativ arbeitende oder direkt positiv arbeitende Emulsionen sein.

Besonders bevorzugt ist ein fotografisches Aufzeichnungsmaterial, in dem mindestens eine der verwendeten Silberhalogenidemulsionen einen Iodidgehalt $\geqq$4 Mol-% und einen mittleren Korndurchmesser $\leqq$0,4 µm hat.

Geeignete Träger zur Herstellung des erfindungsgemäßen Materials sind z.B. Filme und Folien von halbsynthetischen und synthetischen Polymeren, wie Cellulosenitrat, Celluloseacetat, Cellulosebutyrat, Polystyrol, Polyvinylchlorid, Polyethylenterephthalat und Polycarbonat.

Bei dem Silberhalogenid kann es sich um überwiegend kompakte Kristalle handeln, die z.B. regulär kubisch oder oktaedrisch sind oder Übergangsformen aufweisen können. Vorzugsweise können aber auch verzwillingte, z. B. plättchenförmige Kristalle vorliegen, deren durchschnittliches Verhältnis von Durchmesser zu Dicke bevorzugt wenigstens 5:1 ist, wobei der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke größer als 5:1 ist, z.B. 12:1 bis 30:1.

Die Silberhalogenidkörner können auch einen mehrfach geschichteten Kornaufbau aufweisen, im einfachsten Fall mit einem inneren und einem äußeren Kornbereich (core/shell), wobei die Halogenidzusammensetzung und/oder sonstige Modifizierungen, wie z.B. Dotierungen der einzelnen Kornbereiche unterschiedlich sind. Die mittlere Korngröße der Emulsionen liegt vorzugsweise zwischen 0,2 µm und 2,0 µm, die Korngrößenverteilung kann sowohl homo- als auch heterodispers sein. Die Emulsionen können außer dem Silberhalogenid auch organische Silbersalze enthalten, z.B. Silberbenztriazolat oder Silberbehenat.

Es können zwei oder mehrere Arten von Silberhalogenidemulsionen, die getrennt hergestellt werden, als Mischung verwendet werden.

Die fotografischen Emulsionen können nach verschiedenen Methoden (z.B. P. Glafkides, Chimie et Physique Photographique, Paul Montel, Paris (1967), G.F. Duffin, Photographic Emulsion Chemistry, The Focal Press, London (1966), V.L. Zelikman et al, Making and Coating Photographic Emulsion, The Focal Press, London (1966) aus löslichen Silbersalzen und löslichen Halogeniden hergestellt werden.

Die Fällung des Silberhalogenids erfolgt bevorzugt in Gegenwart des Bindemittels, z.B. der Gelatine und kann im sauren, neutralen oder alkalischen pH-Bereich durchgeführt werden, wobei vorzugsweise Silberhalogenidkomplexbildner zusätzlich verwendet werden. Zu letzteren gehören z.B. Ammoniak, Thioether, Imidazol, Ammoniumthiocyanat oder überschüssiges Halogenid. Die Zusammenführung der wasserlöslichen Silbersalze und der Halogenide erfolgt wahlweise nacheinander nach dem single-jet- oder gleichzeitig nach dem double-jet-Verfahren oder nach beliebiger Kombination beider Verfahren. Bevorzugt wird die Dosierung mit steigenden Zuflußraten, wobei die "kritische" Zufuhrgeschwindigkeit, bei der gerade noch keine Neukeime entstehen, nicht überschritten werden sollte. Der pAg-Bereich kann während der Fällung in weiten Grenzen variieren, vorzugsweise wird das sogenannte pAg-gesteuerte Verfahren benutzt, bei dem ein bestimmter pAg-Wert konstant gehalten oder ein definiertes pAg-Profil während der Fällung durchfahren wird. Neben der bevorzugten Fällung bei Halogenidüberschuß ist aber auch die sogenannte inverse Fällung bei Silberionenüberschluß möglich. Außer durch Fällung können die Silberhalogenidkristalle auch durch physikalische Reifung (Ostwaldreifung), in Gegenwart von überschüssigem Halogenid und/oder Silberhalogenidkomplexierungsmittel wachsen. Das Wachstum der Emulsionskörner kann sogar überwiegend durch Ostwaldreifung erfolgen, wobei vorzugsweise eine feinkörnige, sogenannte Lippmann-Emulsion, mit einer schwerer löslichen Emulsion gemischt und auf letzterer umgelöst wird.

Während der Fällung und/oder der physikalischen Reifung der Silberhalogenidkörner können auch Salze oder Komplexe von Metallen, wie Cd, Zn, Pb, Tl, Bi, Ir, Rh, Fe vorhanden sein.

Ferner kann die Fällung auch in Gegenwart von Sensibilisierungsfarbstoffen erfolgen. Komplexierungsmittel und/oder Farbstoffe lassen sich zu jedem beliebigen Zeitpunkt unwirksam machen, z.B. durch Änderung des pH-Wertes oder durch eine oxidative Behandlung.

Als Bindemittel wird vorzugsweise Gelatine verwendet. Diese kann jedoch ganz oder teilweise durch andere synthetische, halbsynthetische oder auch natürlich vorkommende Polymere ersetzt werden. Synthetische Gelatineersatzstoffe sind beispielsweise Polyvinylalkohol, Poly-N-vinylpyrolidon, Polyacrylamide, Polyacrylsäure und deren Derivate, insbesondere deren Mischpolymerisate. Natürlich vorkommende Gelatineersatzstoffe sind beispielsweise andere Proteine wie Albumin oder Casein, Cellulose, Zucker, Stärke oder Alginate. Halbsynthetische Gelatineersatzstoffe sind in der Regel modifizierte Naturprodukte. Cellulosederivate wie Hydroxyalkylcellulose, Carboxymethylcellulose und Phthalylcellulose sowie Gelatinederivate, die durch Umsetzung mit Alkylierungs- oder Acylierungsmitteln oder durch Aufpfropfung von polymerisierbaren Monomeren erhalten worden sind, sind Beispiele hierfür.

Die Bindemittel sollen über eine ausreichende Menge an funktionellen Gruppen verfügen, so daß durch Umsetzung mit geeigneten Härtungsmitteln genügend widerstandsfähigen Schichten erzeugt werden können. Solche funktionellen Gruppen sind insbesondere Aminogruppen, aber auch Carboxylgruppen, Hydroxylgruppen und aktive Methylengruppen.

Die vorzugsweise verwendete Gelatine kann durch sauren oder alkalischen Aufschluß erhalten sein. Die Herstellung solcher Gelatinen wird beispielsweise in The Science and Technology of Gelatine, herausgegeben von A.G. Ward und A. Courts, Academic Press 1977, Seite 295 ff beschrieben. Die jeweils eingesetzte Gelatine soll einen möglichst geringen Gehalt an fotografisch aktiven Verunreinigungen enthalten (Inertgelatine). Gelatinen mit hoher Viskosität und niedriger Quellung sind besonders vorteilhaft. Die Gelatine kann teilweise oder ganz oxidiert sein.

Nach abgeschlossener Kristallbildung oder auch schon zu einem früheren Zeitpunkt werden die löslichen Salze aus der Emulsion entfernt, z.B. durch Nudeln und Waschen, durch Flocken und Waschen, durch Ultrafiltration oder durch Ionenaustauscher.

Die fotografischen Emulsionen können Verbindungen zur Verhinderung der Schleierbildung oder zur Stabilisierung der fotografischen Funktion während der Produktion, der Lagerung oder der fotografischen Verarbeitung enthalten.

Besonders geeignet sind Azaindene, vorzugsweise Tetra- und Pentaazaindene, insbesondere solche, die mit Hydroxyl- oder Aminogruppen substituiert sind. Derartige Verbindungen sind z.B. von Birr, Z. Wiss. Phot. 47 (1952), S. 2 - 58 beschrieben worden. Weiter können als Antischleiermittel Salze von Metallen wie Quecksilber oder Cadmium, aromatische Sulfon- oder Sulfinsäuren wie Benzolsulfinsäure, oder stickstoffhaltige Heterocyclen wie Nitrobenzimidazol, Nitroindazol, (subst.) Benztriazole oder Benzthiazoliumsalze eingesetzt werden. Besonders geeignet sind Mercaptogruppen enthaltende Heterocyclen, z.B. Mercaptobenzthiazole, Mercaptobenzimidazole, Mercaptotetrazole, Mercaptothiadiazole, Mercaptopyrimidine, wobei diese Mercaptoazole auch eine wasserlösliche Gruppe, z.B. eine Carboxylgruppe oder Sulfogruppe, enthalten können. Weitere geeignete Verbindungen sind in Research Disclosure Nr. 17643 (1978), Abschnitt VI, veröffentlicht.

Die Stabilisatoren können den Silberhalogenidemulsionen vor, während oder nach deren Reifung zugesetzt werden. Selbstverständlich kann man die Verbindungen auch anderen fotografischen Schichten, die einer Halogensilberschicht zugeordnet sind, zusetzen.

Es können auch Mischungen aus zwei oder mehreren der genannten Verbindungen eingesetzt werden.

Die Silberhalogenidemulsionen werden üblicherweise chemisch gereift, beispielsweise durch Einwirkung von Goldverbindungen oder Verbindungen des zweiwertigen Schwefels.

Die fotografischen Emulsionsschichten oder andere hydrophile Kolloidschichten des erfindungsgemäß hergestellten lichtempfindlichen Materials können oberflächenaktive Mittel für verschiedene Zwecke enthalten, wie Überzugshilfen, zur Verhinderung der elektrischen Aufladung, zur Verbesserung der Gleiteigenschaften, zum Emulgieren der Dispersion, zur Verhinderung der Adhäsion und zur Verbesserung der fotografischen Charakteristika (z.B. Entwicklungsbeschleunigung, hoher Kontrast, Sensibilisierung usw.).

Geeignete Sensibilisierungsfarbstoffe sind Cyaninfarbstoffe, insbesondere der folgenden Klassen:

1. Rotsensibilisatoren

Dicarbocyanine mit Naphthothiazol oder Benzthiazol als basischen Endgruppen, die in 5- und/oder 6-Stellung durch Halogen, Methyl, Methoxy substituiert sein können sowie 9.11-alkylen-verbrückte, insbesondere 9.11-Neopentylenthiadicarbocyanine mit Alkyl- oder Sulfoalkylsubstituenten am Stickstoff.

2. Grünsensibilisatoren

9-Ethyloxacarbocyanine, die in 5-Stellung durch Chlor oder Phenyl substituiert sind und am Stickstoff der Benzoxazolgruppen Alkyl- oder Sulfoalkylreste, vorzugsweise Sulfoalkylsubstituenten tragen.

3. Blausensibilisatoren

Methincyanine mit Benzoxazol, Benzthiazol, Benzselenazol, Naphthoxazol, Naphthothiazol als basischen Endgruppen, die in 5- und/oder 6-Stellung durch Halogen, Methyl, Methoxy substituiert sein können und mindestens eine, vorzugsweise zwei, Sulfoalkylsubstituenten am Stickstoff tragen. Ferner Apomerocyanine mit einer Rhodaningruppe.

Auf Sensibilisatoren kann verzichtet werden, wenn für einen bestimmten Spektralbereich die Eigenempfindlichkeit des Silberhalogenids ausreichend ist, beispielsweise die Blauempfindlichkeit von Silberbromidiodiden.

Bei einem farbfotografischen Material werden den unterschiedlich sensibilisierten Emulsionsschichten nicht diffundierende monomere oder polymere Farbkuppler zugeordnet, die sich in der gleichen Schicht oder

in einer dazu benachbarten Schicht befinden können. Gewöhnlich werden den rotempfindlichen Schichten Blaugrünkuppler, den grünempfindlichen Schichten Purpurkuppler und den blauempfindlichen Schichten Gelbkuppler zugeordnet.

Farbkuppler zur Erzeugung des blaugrünen Teilfarbenbildes sind in der Regel Kuppler vom Phenol- oder α-Naphtholtyp.

Farbkuppler zur Erzeugung des purpurnen Teilfarbenbildes sind in der Regel Kuppler vom Typ des 5-Pyrazolons, des Indazolons oder der Pyrazoloazole.

Farbkuppler zur Erzeugung des gelben Teilfarbenbildes sind in der Regel Kuppler mit einer offenkettigen Ketomethylengruppierung, insbesondere Kuppler vom Typ des α-Acylacetamids; geeignete Beispiele hierfür sind α-Benzoylacetanilidkuppler und α-Pivaloylacetanilidkuppler.

Bei den Farbkupplern kann es sich um 4-Äquivalentkuppler, aber auch um 2-Äquivalentkuppler handeln. Letztere leiten sich von den 4-Äquivalentkupplern dadurch ab, daß sie in der Kupplungsstelle einen Substituenten enthalten, der bei der Kupplung abgespalten wird.

Die Kuppler enthalten üblicherweise einen Ballastrest, um eine Diffusion innerhalb des Materials, d.h. sowohl innerhalb einer Schicht oder von Schicht zu Schicht, unmöglich zu machen. Anstelle von Kupplern mit einem Ballastrest können auch hochmolekulare Kuppler eingesetzt werden.

Geeignete Farbkuppler bzw. Literaturstellen, in denen solche beschrieben sind, finden sich z.B. in Research Disclosure 17 643 (1978), Kapitel VII.

Hochmolekulare Farbkuppler sind beispielsweise in DE-C-1 297 417, DE-A-24 07 569, DE-A-31 48 125, DE-A-32 17 200, DE-A-33 20 079, DE-A-33 24 932, DE-A-33 31 743, DE-A-33 40 376, EP-A-27 284, US-A-4 080 211 beschrieben. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Das Material kann weiterhin Verbindungen enthalten, die eine fotografisch wirksame Substanz in Freiheit setzen können, beispielsweise einen Entwicklungsinhibitor, einen Entwicklungsbeschleuniger, einen Bleichbeschleuniger, einen Entwickler oder ein Schleiermittel.

Beispiele für einen Entwicklungsinhibitor abspaltende Verbindungen sind DIR-Kuppler (siehe z.B. Research Disclosure 17 643 (1978), Kapitel VII F) und IRD-Verbindungen (siehe z.B. US-A-4 684 604 und DE-A-31 45 640).

Beispiele für einen Entwicklungsbeschleuniger oder ein Schleiermittel abspaltende Verbindungen sind die sogenannten DAR- bzw. FAR-Kuppler (siehe z.B. DE-A-25 34 466, 34 41 823 und EP-A-0 147 765).

Beispiele für einen Bleichbeschleuniger abspaltende Verbindungen sind die sogenannten BAR-Kuppler (siehe z.B. EP-A-0 193 389).

Die Einarbeitung der Kuppler oder anderer Verbindungen in Silberhalogenidemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittels hängt von der jeweiligen Löslichkeit der Verbindung ab.

Methoden zum Einbringen von in Wasser im wesentlichen unlöslichen Verbindungen durch Mahlverfahren sind beispielsweise in DE-A-26 09 741 und DE-A-26 09 742 beschrieben.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 027, US-A-2 801 170, US-A-2 801 171 und EP-A-0 043 037 beschrieben.

Anstelle der hochsiedenden Lösungsmittel können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113.

Die diffusionsfeste Einlagerung anionischer wasserlöslicher Verbindungen (z.B. von Farbstoffen) kann auch mit Hilfe von kationischen Polymeren, sogenannten Beizenpolymeren erfolgen.

Geeignete Ölbildner sind z.B. Phthalsäurealkylester, Phosphonsäureester, Phosphorsäureester, Citronensäureester, Benzoesäureester, Amide, Fettsäureester, Trimesinsäureester, Alkohole, Phenole, Anilinderivate und Kohlenwasserstoffe.

Beispiele für geeignete Ölbildner sind Dibutylphthalat, Dicyclohexylphthalat, Di-2-ethylhexylphthalat, Decylphthalat, Triphenylphosphat, Tricresylphosphat, 2-Ethylhexyldiphenylphosphat, Tricyclohexylphosphat, Tri-2-ethylhexylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-tert.-amylphenol, Dioctylacelat, Glycerintributyrat, Isostearyllactat, Trioctylcitrat, N,N-Dibutyl-2-butoxy-5-tert.-octylanilin, Paraffin, Dodecylbenzol und Diisopropylnaphthalin.

Das fotografische Material kann weiterhin UV-Licht absorbierende Verbindungen, Weißtöner, Abstandshalter, Filterfarbstoffe, Formalinfänger, Lichtschutzmittel, Antioxidantien, $D_{Min}$-Farbstoffe, Zusätze zur Verbesserung der Farbstoff-, Kuppler- und Weißenstabilisierung sowie zur Verringerung des Farbschleiers, Weichmacher (Latices), Biocide und anderes enthalten.

UV-Licht absorbierende Verbindungen sollen einerseits die Bildfarbstoffe vor dem Ausbleichen durch UV-reiches Tageslicht schützen und andererseits als Filterfarbstoffe das UV-Licht im Tageslicht bei der Belichtung absorbieren und so die Farbwiedergabe eines Films verbessern. Üblicherweise werden für die beiden Aufgaben Verbindungen unterschiedlicher Struktur eingesetzt. Beispiele sind arylsubstituierte Benzotriazolverbindungen (US-A-3 533 794), 4-Thiazolidonverbindungen (US-A-3 314 794 und 3 352 681), Benzophenonverbindungen (JP-A-2784/71), Zimtsäureesterverbindungen (US-A-3 705 805 und 3 707 375), Butadienverbindungen (US-A-4 045 229) oder Benzoxazolverbindungen (US-A-3 700 455).

Es können auch ultraviolettabsorbierende Kuppler (wie Blaugrünkuppler des $\alpha$-Naphtholtyps) und ultraviolettabsorbierende Polymere verwendet werden. Diese Ultraviolettabsorbentien können durch Beizen in einer speziellen Schicht fixiert sein.

Für sichtbares Licht geeignete Filterfarbstoffe umfassen Oxonolfarbstoffe, Hemioxonolfarbstoffe, Styrylfarbstoffe, Merocyaninfarbstoffe, Cyaninfarbstoffe und Azofarbstoffe. Von diesen Farbstoffen werden Oxonolfarbstoffe, Hemioxonolfarbstoffe und Merocyaninfarbstoffe besonders vorteilhaft verwendet.

Geeignete Weißtöner sind z.B. in Research Disclosure 17 643 (Dez. 1978), Kapitel V, in US-A-2 632 701, 3 269 840 und in GB-A-852 075 und 1 319 763 beschrieben.

Bestimmte Bindemittelschichten, insbesondere die vom Träger am weitesten entfernte Schicht, aber auch gelegentlich Zwischenschichten, insbesondere, wenn sie während der Herstellung die vom Träger am weitesten entfernte Schicht darstellen, können fotografisch inerte Teilchen anorganischer oder organischer Natur enthalten, z.B. als Mattierungsmittel oder als Abstandshalter (DE-A-33 31 542, DE-A-34 24 893, Research Disclosure 17 643, (Dez. 1978), Kapitel XVI).

Der mittlere Teilchendurchmesser der Abstandshalter liegt insbesondere im Bereich von 0,2 bis 10 µm. Die Abstandshalter sind wasserunlöslich und können alkaliunlöslich oder alkalilöslich sein, wobei die alkalilöslichen im allgemeinen im alkalischen Entwicklungsbad aus dem fotografischen Material entfernt werden. Beispiele für geeignete Polymere sind Polymethylmethacrylat, Copolymere aus Acrylsäure und Methylmethacrylat sowie Hydroxypropylmethylcellulosehexahydrophthalat.

Zusätze zur Verbesserung der Farbstoff-, Kuppler- und Weißenstabilität sowie zur Verringerung des Farbschleiers (Research Disclosure 17 643/1978, Kapitel VII) können den folgenden chemischen Stoffklasen angehören: Hydrochinone, 6-Hydroxychromane, 5-Hydroxycumarane, Spirochromane, Spiroindane, p-Alkoxyphenole, sterisch gehinderte Phenole, Gallussäurederivate, Methylendioxybenzole, Aminophenole, sterisch gehinderte Amine, Derivate mit veresterten oder verätherten phenolischen Hydroxylgruppen, Metallkomplexe.

Verbindungen, die sowohl eine sterisch gehinderte Amin-Partialstruktur als auch eine sterisch gehinderte Phenol-Partialstruktur in einem Molekül aufweisen (US-A-4 268 593), sind besonders wirksam zur Verhinderung der Beeinträchtigung (Verschlechterung bzw. Abbau) von gelben Farbbildern als Folge der Entwicklung von Wärme, Feuchtigkeit und Licht. Um die Beeinträchtigung (Verschlechterung bzw. den Abbau) von purpurroten Farbbildern, insbesondere ihre Beeinträchtigung (Verschlechterung bzw. Abbau) als Folge der Einwirkung von Licht, zu verhindern, sind Spiroindane (JP-A-159 644/81) und Chromane, die durch Hydrochinondiether oder -monoether substiutiert sind (JP-A-89 835/80) besonders wirksam.

Die Schichten des fotografischen Materials können mit den üblichen Härtungsmitteln gehärtet werden. Geeignete Härtungsmittel sind z.B. Formaldehyd, Glutaraldehyd und ähnliche Aldehydverbindungen, Diacetyl, Cyclopentadion und ähnliche Ketonverbindungen, Bis-(2-chlorethylharnstoff), 2-Hydroxy-4,6-dichlor-1,3,5-triazin und andere Verbindungen, die reaktives Halogen enthalten (US-A-3 288 775, US-A-2 732 303, GB-A-974 723 und GB-A-1 167 207) Divinylsulfonverbindungen, 5-Acetyl-1,3-diacryloylhexahydro-1,3,5-triazin und andere Verbindungen, die eine reaktive Olefinbindung enthalten (US-A-3 635 718, US-A-3 232 763 und GB-A-994 869); N-Hydroxymethylphthalimid und andere N-Methylolverbindungen (US-A-2 732 316 und US-A-2 586 168); Isocyanate (US-A-3 103 437); Aziridinverbindungen (US-A-3 017 280 und US-A-2 983 611); Säurederivate (US-A-2 725 294 und US-A-2 725 295); Verbindungen vom Carbodiimidtyp (US-A-3 100 704); Carbamoylpyridiniumsalze (DE-A-22 25 230 und DE-A-24 39 551); Carbamoyloxypyridiniumverbindungen (DE-A-24 08 814); Verbindungen mit einer Phosphor-Halogen-Bindung (JP-A-113 929/83); N-Carbonyloximid-Verbindungen (JP-A-43353/81); N-Sulfonyloximido-Verbindungen (US-A-4 111 926), Dihydrochinolinverbindungen (US-A-4 013 468), 2-Sulfonyloxypyridiniumsalze (JP-A-110 762/81), Formamidiniumsalze (EP-A-0 162 308), Verbindungen mit zwei oder mehr N-Acyloximino-Gruppen (US-A-4 052 373), Epoxyverbindungen (US-A-3 091 537), Verbindungen vom Isoxazoltyp (US-A-3 321 313 und US-A-3 543 292); Halogencarboxyaldehyde, wie Mucochlorsäure; Dioxanderivate, wie Dihydroxydioxan und Di-chlordioxan; und

anorganische Härter, wie Chromalaun und Zirkonsulfat.

Die Härtung kann in bekannter Weise dadurch bewirkt werden, daß das Härtungsmittel der Gießlösung für die zu härtende Schicht zugesetzt wird, oder dadurch, daß die zu härtende Schicht mit einer Schicht überschichtet wird, die ein diffusionsfähiges Härtungsmittel enthält.

Unter den aufgeführten Klassen gibt es langsam wirkende und schnell wirkende Härtungsmittel sowie sogenannte Soforthärter, die besonders vorteilhaft sind. Unter Soforthärtern werden Verbindungen verstanden, die geeignete Bindemittel so vernetzen, daß unmittelbar nach Beguß, spätestens nach 24 Stunden, vorzugsweise spätestens nach 8 Stunden die Härtung so weit abgeschlossen ist, daß keine weitere durch die Vernetzungsreaktion bedingte Änderung der Sensitometrie und der Quellung des Schichtverbandes auftritt. Unter Quellung wird die Differenz von Naßschichtdicke und Trockenschichtdicke bei der wäßrigen Verarbeitung des Films verstanden (Photogr. Sci., Eng. 8 (1964), 275; Photogr. Sci. Eng. (1972), 449).

Bei diesen mit Gelatine sehr schnell reagierenden Härtungsmitteln handelt es sich z.B. um Carbamoylpyridiniumsalze, die mit freien Carboxylgruppen der Gelatine zu reagieren vermögen, so daß letztere mit freien Aminogruppen der Gelatine unter Ausbildung von Peptidbindungen und Vernetzung der Gelatine reagieren.

Es gibt diffusionsfähige Härtungsmittel, die auf alle Schichten innerhalb eines Schichtverbandes in gleicher Weise härtend wirken. Es gibt aber auch schichtbegrenzt wirkende, nicht diffundierende, niedermolekulare und hochmolekulare Härter. Mit ihnen kann man einzelnen Schichten, z.B. die Schutzschicht besonders stark vernetzen. Dies ist wichtig, wenn man die Silberhalogenid-Schicht wegen der Silberdeckkrafterhöhung wenig härtet und mit der Schutzschicht die mechanischen Eigenschaften verbessern muß (EP-A 0 114 699).

Die erfindungsgemäßen schwarzweißfotografischen Materialien werden üblicherweise durch Entwickeln, Fixieren und Wässern oder Stabilisieren ohne nachfolgende Wässerung verarbeitet. Im Fall einer Umkehrentwicklung gehen diesem Prozeß eine Erstentwicklung mit nachfolgendem Bleichschritt und eine diffuse Zweitbelichtung oder eine chemische Verschleierung voraus. Als Entwicklersubstanzen werden Reduktionsmittel wie Phenole, Phenolamine und Pyrazolinone eingesetzt. Für die Schwarzweißfotografie besonders geeignet sind z.B. Hydrochinon, Metol und Phenidon.

Die erfindungsgemäßen farbfotografischen Materialien werden üblicherweise durch Entwickeln, Bleichen, Fixieren und Wässern oder Stabilisieren ohne nachfolgende Wässerung verarbeitet, wobei Bleichen und Fixieren zu einem Verarbeitungsschritt zusammengefaßt sein können. Bei einer Umkehrentwicklung gehen der Farbentwicklung eine Erstentwicklung mit einem Entwickler, der mit den Kupplern keinen Farbstoff bildet, und eine diffuse Zweitbelichtung oder eine chemische Verschleierung voraus.

Als Farbentwicklerverbindung lassen sich sämtliche Entwicklerverbindungen verwenden, die die Fähigkeit besitzen, in Form ihres Oxidationsproduktes mit Farbkupplern zu Azomethin- bzw. Indophenolfarbstoffen zu reagieren. Geeignete Farbentwicklerverbindungen sind aromatische, mindestens eine primäre Aminogruppe enthaltende Verbindungen vom p-Phenylendiamintyp, beispielsweise N,N-Dialkyl-p-phenylendiamine wie N,N-Diethyl-p-phenylendiamin, 1-(N-Ethyl-N-methansulfonamidoethyl)-3-methyl-p-phenylendiamin, 1-(N-Ethyl-N-hydroxyethyl)-3-methyl-p-phenylendiamin und 1-(N-Ethyl-N-methoxyethyl)-3-methyl-p-phenylendiamin. Weitere brauchbare Farbentwickler sind beispielsweise in J. Amer. Chem. Soc. 73, 3106 (1951) und G. Haist, Modern Photographic Processing, 1979, John Wiley and Sons, New York, Seite 545 ff. beschrieben.

Nach der Farbentwicklung kann ein saures Stoppbad oder eine Wässerung folgen. entsprechenden Phosphonsäuren. Geeignet als Bleichmittel sind weiterhin Persulfate und Peroxide, z.B. Wasserstoffperoxid.

Auf das Bleichfixierbad oder Fixierbad folgt meist eine Wässerung, die als Gegenstromwässerung ausgeführt ist oder aus mehreren Tanks mit eigener Wasserzufuhr besteht.

Günstige Ergebnisse können bei Verwendung eines darauf folgenden Schlußbades, das keinen oder nur wenig Formaldehyd enthält, erhalten werden.

Die Wässerung kann aber durch ein Stabilisierbad vollständig ersetzt werden, das üblicherweise im Gegenstrom geführt wird. Dieses Stabilisierbad übernimmt bei Formaldehydzusatz auch die Funktion eines Schlußbades.

Beispiel 1

Aufzeichnungsmaterial 1.1

Auf einen mit einer Haftschicht versehenen Schichtträger aus Cellulosetriacetat wurden nacheinander die im folgenden aufgeführten Schichten aufgetragen.

1. Schicht (Emulsionsschicht)

| | |
|---|---|
| Silberhalogenidemulsion (mittl. Korndurchmesser 0,22 µm, 94 Mol-% Bromid, 6 Mol-% Iodid) | |
| Silberauftrag als Silbernitrat | 2,50 $g/m^2$ |
| Kuppler C-1 | 2,00 $g/m^2$ |
| Gelatine | 5,50 $g/m^2$ |
| Trikresylphosphat (TKP) | 1,00 $g/m^2$ |

2. Schicht (Schutzschicht)

| | |
|---|---|
| Gelatine | 1,80 $g/m^2$ |
| Härtungsmittel H | 1,08 $g/m^2$ |

Die Vergleichsmaterialien 1.6 bis 1.9 und die erfindungsgemäßen Materialien 1.2 bis 1.5 wurden in der Weise erhalten, daß jeweils 12,5 µmol/$m^2$ der in Tabelle 2 angegebenen Verbindungen zu Schicht 1 zugegeben wurden. Proben der so hergestellten Materialien wurden nach Belichtung einer Colorumkehrentwicklung unterzogen, wie beschrieben in "Manual for PROCESSING Kodak Ektachrome Film using Process E7", Eastman Kodak Company, 1977 (vergleiche Kodak Publikation Nr. Z-119).

Der angegebene Kanteneffekt ist die Differenz zwischen Mikrodichte (Spaltbreite 30 µm) und Makrodichte umkehrentwickelter Proben, wie beschrieben in T.H. James, The Theory of the Photographic Process, 4. Auflage, Macmillan Publishing Co., Inc., S. 611 (1977).

Die so ermittelten Versuchsdaten sind in Tabelle 2 angegeben.

Tabelle 2

| Nr. | Verbindung | Kanteneffekt δ D bei der Makrodichte D=0,6 | D=1,0 | D=1,4 | |
|---|---|---|---|---|---|
| 1.1 | - | -0,13 | -0,14 | -0,14 | Referenz ohne Zusatz |
| 1.2 | B-1 | -0,41 | -0,56 | -0,62 | |
| 1.3 | B-2 | -0,37 | -0,49 | -0,51 | |
| 1.4 | B-3 | -0,43 | -0,55 | -0,55 | |
| 1.5 | B-4 | -0,38 | -0,49 | -0,50 | |
| 1.6 | V-1 | -0,01 | -0,14 | -0,21 | Vergleich |
| 1.7 | V-2 | -0,17 | -0,23 | -0,24 | Vergleich |
| 1.8 | V-3 | -0,04 | -0,08 | -0,16 | Vergleich |
| 1.9 | V-4 | -0,32 | -0,37 | -0,35 | Vergleich |

Wie aus Tabelle 2 ersichtlich ist, verstärken die erfindungsgemäßen Verbindungen den Kanteneffekt in erheblichem Maß, während die Vergleichsverbindungen keine oder nur kleine Effekte zeigen.

Beispiel 2

Aufzeichnungsmaterial 2.1

Auf einen mit einer Haftschicht versehenen Schichtträger aus Cellulosetriacetat wurden nacheinander die im folgenden aufgeführten Schichten aufgetragen.

1. Schicht (rotempfindliche Schicht)

| | |
|---|---|
| rotsensibilisierte Silberhalogenidemulsion (mittl. Korndurchmesser 0,22 µm, 94 Mol-% Bromid, 6 Mol-% Iodid) | |
| Silberauftrag als Silbernitrat | 2,50 $g/m^2$ |
| Kuppler C-1 | 2,00 $g/m^2$ |
| Gelatine | 3,00 $g/m^2$ |
| TKP | 1,00 $g/m^2$ |

2. Schicht (Zwischenschicht)

| | |
|---|---|
| Gelatine | 2,15 $g/m^2$ |
| Verbindung S | 0,24 $g/m^2$ |
| TKP | 0,12 $g/m^2$ |

3. Schicht (grünempfindliche Schicht)

| | |
|---|---|
| grünsensibilisierte Silberhalogenidemulsion (mittl. Korndurchmesser 0,22 µm, 94 Mol-% Bromid, 6 Mol-% Iodid) | |
| Silberauftrag als Silbernitrat | 2,50 $g/m^2$ |
| Kuppler C-2 | 1,50 $g/m^2$ |
| Gelatine | 2,60 $g/m^2$ |
| TKP | 0,75 $g/m^2$ |

4. Schicht (Schutzschicht)

| | |
|---|---|
| Gelatine | 1,80 $g/m^2$ |
| Härtungsmittel H | 1,08 $g/m^2$ |

Die Testaufbauten 2.2 bis 2.8 wurden dadurch erhalten, daß je 0,5 mmol der in der Tabelle angegebenen Verbindung pro 100 g Emulsion (gerechnet als Silbernitrat) zu den in der Tabelle angegebenen Schichten zugegeben wurde. Material 2.9 wurde dadurch erhalten, daß in der 3. Schicht die angegebene Emulsion durch eine Emulsion mit mittlerem Korndurchmesser 0,34 µm, 96 Mol-% Bromid und 4 Mol-% Iodid ersetzt wurde.

Der Kanteneffekt wurde bestimmt wie in Beispiel 1 angegeben. Der Interimage-Effekt Rot ist die Empfindlichkeitsdifferenz bei D=1,0 der rotempfindlichen Schicht in einer Selektivbelichtung Rot gegenüber einer Additivbelichtung Rot + Grün derart, daß bei Additivbelichtung Rot- und Grünempfindlichkeit gleich waren. Entsprechendes gilt für den Interimage-Effekt Grün.

Tabelle 3

| Nr. | Verb. | Schicht | Kanteneffekt δD(bei Makrodichte D=1,0) | | Interimage-Effekt δE(D=1,0) | |
|---|---|---|---|---|---|---|
| | | | bg | pp | Rot | Grün |
| 2.1 | - | - | -0,10 | -0,15 | 0,2 | 0,2 |
| 2.2 | B-2 | 1+3 | -0,58 | -0,63 | 1,6 | 1,4 |
| 2.3 | B-1 | 1+3 | -0,72 | -0,79 | 2,2 | 1,2 |
| 2.4 | V-5 | 1+3 | -0,49 | -0,48 | 0,6 | 0,5 |
| 2.5 | V-6 | 1+3 | -0,20 | -0,57 | 0,0 | 0,4 |
| 2.6 | V-7 | 1+3 | -0,41 | -0,50 | 0,6 | 0,8 |
| 2.7 | V-8 | 1+3 | -0,20 | -0,20 | 0,2 | 0,2 |
| 2.8 | B-2 | 1 | -0,41 | -0,38 | 2,2 | 0,3 |
| 2.9 | - | - | -0,04 | -0,07 | 0,0 | 0,0 |

Wie aus der Tabelle ersichtlich, verstärken die erfindungsgemäßen Substanzen den Kanten- und Interimage-Effekt.

Beispiel 3

Aufzeichnungsmaterial 3.1

Auf einen mit einer Haftschicht versehenen Schichtträger aus Cellulosetriacetat wurden nacheinander die im folgenden aufgeführten Schichten aufgetragen.

1. Schicht (Antihaloschicht)

| | |
|---|---|
| schwarzes kolloidales Silbersol | 0,25 g/$m^2$ |
| Gelatine | 1,60 g/$m^2$ |
| UV-Absorber UV | 0,24 g/$m^2$ |

2. Schicht (Zwischenschicht)

| | |
|---|---|
| Gelatine | 0,64 g/$m^2$ |

3. Schicht (erste rotempfindliche Schicht)

rotsensibilisierte Silberhalogenidemulsion (mittl. Korndurchmesser 0,34 µm, 96 Mol-% Bromid, 4 Mol-% Iodid)

| | |
|---|---|
| Silberauftrag als Silbernitrat | 0,95 g/$m^2$ |
| Kuppler C-1 | 0,24 g/$m^2$ |
| Gelatine | 0,80 g/$m^2$ |
| TKP | 0,12 g/$m^2$ |

4. Schicht (zweite rotempfindliche Schicht)

rotsensibilisierte Silberhalogenidemulsion (mittl. Korndurchmesser 0,43 µm, 97 Mol-% Bromid, 3 Mol-% Iodid)

| | |
|---|---|
| Silberauftrag als Silbernitrat | 2,00 g/$m^2$ |
| Kuppler C-1 | 1,29 g/$m^2$ |
| Gelatine | 2,64 g/$m^2$ |
| TKP | 0,65 g/$m^2$ |

5. Schicht (Zwischenschicht)

| | |
|---|---|
| Gelatine | 1,78 g/$m^2$ |
| Verbindung S | 0,24 g/$m^2$ |
| TKP | 0,12 g/$m^2$ |

6. Schicht (erste grünempfindliche Schicht)

| | |
|---|---|
| grünsensibilisierte Silberhalogenidemulsion (mittl. Korndurchmesser 0,34 µm, 96 Mol-% Bromid, 4 Mol-% Iodid) | |
| Silberauftrag als Silbernitrat | 1,05 g/m$^2$ |
| Kuppler C-3 | 0,22 g/m$^2$ |
| Gelatine | 1,00 g/m$^2$ |
| TKP | 0,22 g/m$^2$ |

7. Schicht (zweite grünempfindliche Schicht)

| | |
|---|---|
| grünsensibilisierte Silberhalogenidemulsion (mittl. Korndurchmesser 0,42 µm, 98,5 Mol-% Bromid, 1,5 Mol-% Iodid) | |
| Silberauftrag als Silbernitrat | 1,65 g/m$^2$ |
| Kuppler C-3 | 1,00 g/m$^2$ |
| Gelatine | 2,65 g/m$^2$ |
| TKP | 1,00 g/m$^2$ |

8. Schicht (Zwischenschicht)

| | |
|---|---|
| Gelatine | 0,70 g/m$^2$ |
| Verbindung S | 0,10 g/m$^2$ |
| TKP | 0,05 g/m$^2$ |

9. Schicht (Filtergelbschicht)

| | |
|---|---|
| gelbes kolloidales Silbersol | |
| Silberauftrag als Silbernitrat | 0,19 g/m$^2$ |
| Gelatine | 0,75 g/m$^2$ |

10. Schicht (Zwischenschicht)

| | |
|---|---|
| Gelatine | 0,50 g/m$^2$ |

11. Schicht (erste blauempfindliche Schicht)

| | |
|---|---|
| blausensibilisierte Silberhalogenidemulsion (mittl. Korndurchmesser 0,52 µm, 95 Mol-% Bromid, 5 Mol-% Iodid) | |
| Silberauftrag als Silbernitrat | 0,60 g/m$^2$ |
| Kuppler C-2 | 0,60 g/m$^2$ |
| Gelatine | 0,90 g/m$^2$ |
| TKP | 0,30 g/m$^2$ |

12. Schicht (zweite blauempfindliche Schicht)

| | |
|---|---|
| blausensibilisierte Silberhalogenidemulsion (mittl. Korndurchmesser 0,70 µm, 95 Mol-% Bromid, 5 Mol-% Iodid) | |
| Silberauftrag als Silbernitrat | 0,90 g/m² |
| Kuppler C-2 | 0,90 g/m² |
| Gelatine | 1,00 g/m² |
| TKP | 0,45 g/m² |

13. Schicht (Zwischenschicht)

| | |
|---|---|
| Verbindung S | 0,50 g/m² |
| Gelatine | 2,56 g/m² |
| TKP | 0,02 g/m² |
| UV-Absorber UV | 0,55 g/m² |

14. Schicht (Zwischenschicht)

| | |
|---|---|
| Silberhalogenidemulsion vom Lippmann-Typ (mittl. Korndurchmesser 0,15 µm, 96 Mol-% Bromid, 4 Mol-% Iodid) | |
| Silberauftrag als Silbernitrat | 0,33 g/m² |
| Gelatine | 0,60 g/m² |

15. Schicht (Schutzschicht)

| | |
|---|---|
| Härtungsmittel H | 1,20 g/m² |
| Gelatine | 0,80 g/m² |

Die Proben 3.2 bis 3.4 wurden dadurch erhalten, daß wie in Tabelle 4 angegeben die Verbindung B-2 zugegeben wurde.

Der Kanteneffekt wurde bestimmt wie in Beispiel 1 beschrieben. Der Interimage-Effekt Rot ist die Empfindlichkeitsdifferenz bei D=1,5 der rotempfindlichen Schicht in einer Selektivbelichtung Rot gegenüber einer Weißbelichtung (erhalten durch Additivbelichtung Rot + Grün + Blau). Entsprechendes gilt für die Interimage-Effekte Grün und Blau.

Die Körnigkeit ist die bei Makrodichte 1,0 gemessene Standardabweichung $\sigma_D$ einer Mikrodensitometerspur wie beschrieben in T.H. James, The Theory of the Photographic Process, 4. Auflage, Macmillan Publishing Co., Inc., S. 618 (1977) (Meßbedingungen: Paralleles Licht, Meßblende 28,9 µm), Die Schärfe (MTF) wurde ermittelt wie beschrieben in T.H. James, The Theory of the Photographic Process, 4. Auflage, Macmillan Publishing Co., Inc., S. 604 (1977).

Tabelle 4

| Nr. | Verb. | [µg/m²] Schicht 3 | [µg/m²] Schicht 6 | Kanteneffekt δD (bei Makrodichte D=1,0) bg | Kanteneffekt δD (bei Makrodichte D=1,0) pp | Interimage-Effekt δE(D=1,5) Rot | Interimage-Effekt δE(D=1,5) Grün | Interimage-Effekt δE(D=1,5) Blau | Körnigkeit x $10^3$ $\sigma_D$(D=1,0) bg | Körnigkeit x $10^3$ $\sigma_D$(D=1,0) pp | Schärfe[%] 10 Lin/mm bg | Schärfe[%] 10 Lin/mm pp |
|---|---|---|---|---|---|---|---|---|---|---|---|---|
| 3.1 | - | - | - | -0,34 | -0,23 | +0,2 | -0,8 | -1,0 | 34 | 30 | 88 | 96 |
| 3.2 | B-2 | 950 | - | -0,42 | -0,42 | 1,3 | 0,3 | -1,0 | 32 | 30 | 93 | 98 |
| 3.3 | B-2 | - | 530 | -0,45 | -0,48 | 0,6 | 0,5 | -1,0 | 29 | 27 | 89 | 103 |
| 3.4 | B-2 | 950 | 530 | -0,50 | -0,47 | 1,3 | 1,2 | -1,0 | 28 | 25 | 92 | 103 |

Wie aus der Tabelle ersichtlich wird durch die erfindungsgemäße Verbindung Kanteneffekt, Interimage-Effekt, Schärfe und Körnigkeit verbessert.

Die verwendeten Komponenten haben folgende Formel:

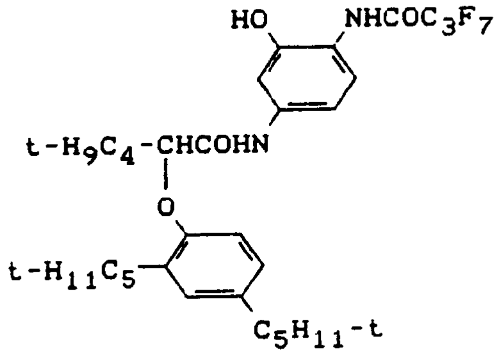

C-1

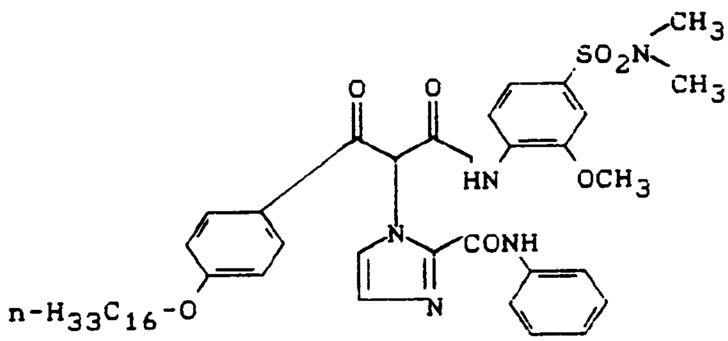

C-2

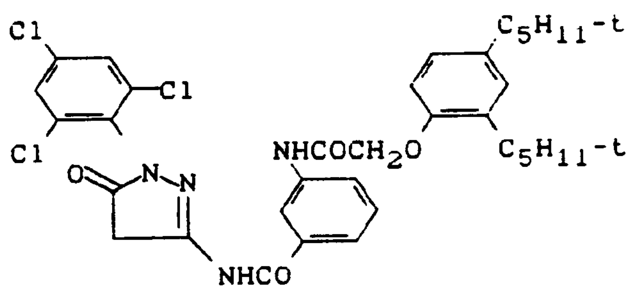

C-3

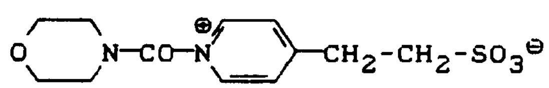

H

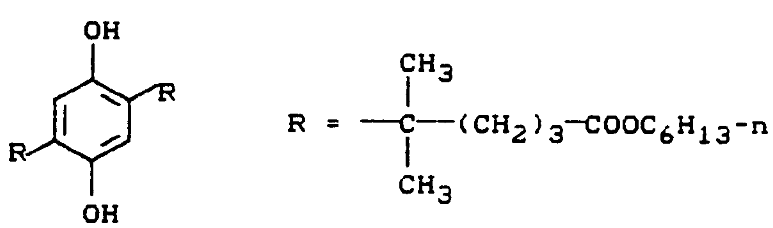

S

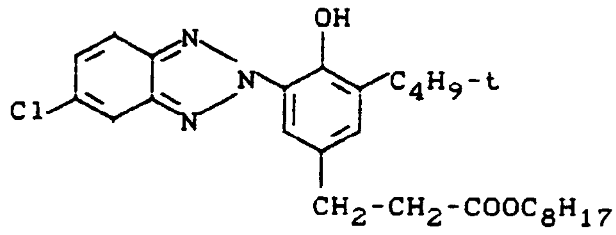

UV

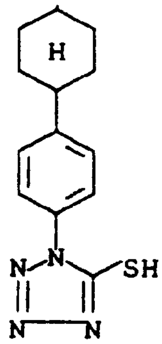

V-1

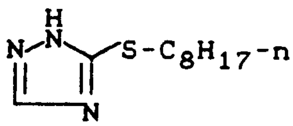

V-2

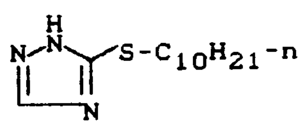

V-3

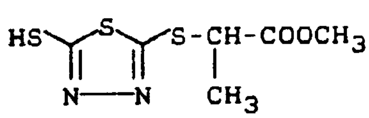

V-4

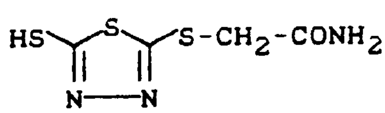

V-5

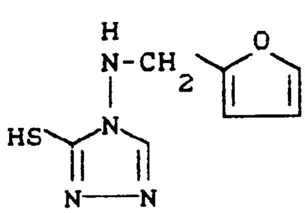

V-6

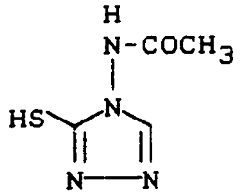

V-7

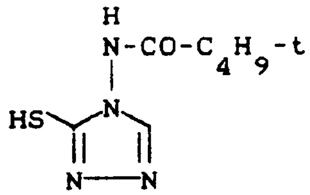

V-8

## Patentansprüche

1. Fotografisches Aufzeichnungsmaterial enthaltend einen transparenten Träger mit mindestens einer lichtempfindlichen Silberhalogenidemulsionsschicht sowie gegebenenfalls üblichen Zwischen- und Schutzschichten, dadurch gekennzeichnet, daß das fotografische Aufzeichnungsmaterial in mindestens einer der genannten Schichten eine Verbindung der Formel I

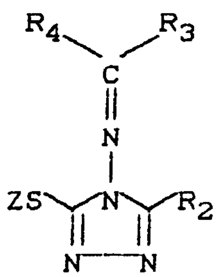

(I)

enthält, worin

$R_2$ Wasserstoff, einen unsubstituierten oder substituierten Alkylrest, einen unsubstituierten oder substituierten Arylrest, einen unsubstituierten oder substituierten Alkylthiorest,

$R_3$ Wasserstoff, einen unsubstituierten oder substituierten Alkylrest,

$R_4$ einen unsubstituierten oder substituierten Alkylrest, einen unsubstituierten oder substituierten Arylrest, einen unsubstituierten oder substituierten heterocyclischen Rest,

Z Wasserstoff, ein Kation oder eine Vorläuferverbindung davon, die bei der Entwicklung nichtbildmäßig die Mercaptoverbindung freisetzen kann,

bedeuten.

2. Fotografisches Aufzeichnungsmaterial nach Anspruch 1, dadurch gekennzeichnet, daß es die Verbindung in einer lichtempfindlichen Silberhalogenidemulsionsschicht enthält.

3. Fotografisches Aufzeichnungsmaterial nach einem der Ansprüche 1 oder 2, dadurch gekennzeichnet, daß es sich um ein farbfotografisches Aufzeichnungsmaterial mit mindestens einer rotempfindlichen, mindestens einer grünempfindlichen und mindestens einer blauempfindlichen Silberhalogenidemulsionsschicht, die in der angegebenen Reihenfolge mindestens einen Blaugrünkuppler, mindestens einen Purpurkuppler und mindestens einen Gelbkuppler enthalten, handelt.

4. Fotografisches Aufzeichnungsmaterial nach einem der Ansprüche 1 bis 3, dadurch gekennzeichnet, daß es sich um ein Colorumkehrmaterial handelt.

5. Fotografisches Aufzeichnungsmaterial nach einem der Ansprüche 1 bis 4, dadurch gekennzeichnet, daß mindestens eine der verwendeten Silberhalogenidemulsionsschichten einen Iodidgehalt ≧ 4 Mol-% und einen mittleren Korndurchmesser ≦ 0,4 µm hat.

## Claims

1. Photographic recording material containing a transparent support with at least one photosensitive silver halide emulsion layer and optionally customary interlayers and protective layers, characterised in that the photographic recording material contains in at least one of the stated layers a compound of the formula I

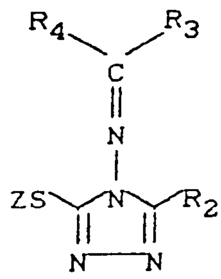

(I)

in which

$R_2$ means hydrogen, an unsubstituted or substituted alkyl residue, an unsubstituted or substituted aryl residue, an unsubstituted or substituted alkylthio residue,

$R_3$ means hydrogen, an unsubstituted or substituted alkyl residue,

$R_4$ means an unsubstituted or substituted alkyl reside, an unsubstituted or substituted aryl residue, an unsubstituted or substituted heterocyclic residue,

Z means hydrogen, a cation or a precursor compound thereof, which is capable of releasing the mercapto compound non-imagewise in the course of development.

2. Photographic recording material according to claim 1, characterised in that it contains the compound in a photosensitive silver halide emulsion layer.

3. Photographic recording material according to one of claims 1 or 2, characterised in that it is a colour photographic recording material comprising at least one red-sensitive, at least one green-sensitive and at least one blue-sensitive silver halide emulsion layer which contain in the stated order at least one cyan coupler, at least one magenta coupler and at least one yellow coupler.

4. Photographic recording material according to one of claims 1 to 3, characterised in that it is a colour reversal material.

5. Photographic recording material according to one of claims 1 to 4, characterised in that at least one of the silver halide emulsion layers used has an iodide content of ≧ 4 mol % and an average grain diameter of ≦ 0.4 µm.

## Revendications

1. Matériau d'enregistrement photographique contenant un support transparent avec au moins une couche photosensible d'émulsion à l'halogénure d'argent, de même que éventuellement des couches intermédiaires et protectrices habituelles, caractérisé en ce que le matériau d'enregistrement photographique contient, dans au moins une des couches mentionnées, un composé de formule I

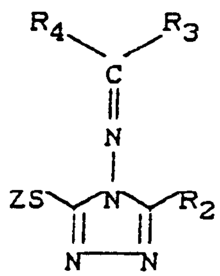

(I)

dans laquelle

R2 représente un atome d'hydrogène, un radical alkyle non substitué ou substitué, un radical aryle non substitué ou substitué, un radical alkylthio non substitué ou substitué,

R3 représente un atome d'hydrogène, un radical alkyle non substitué ou substitué,

R4 représente un radical alkyle non substitué ou substitué, un radical aryle non substitué ou substitué, un radical hétérocyclique non substitué ou substitué,

Z représente un atome d'hydrogène, un cation ou un composé précurseur de ce dernier, qui peut libérer le composé mercapto lors du développement non en forme d'image.

**2.** Matériau d'enregistrement photographique selon la revendication 1, caractérisé en ce qu'il contient le composé dans une couche photosensible d'émulsion à l'halogénure d'argent.

**3.** Matériau d'enregistrement photographique selon l'une quelconque des revendications 1 ou 2, caractérisé en ce qu'il s'agit d'un matériau d'enregistrement pour photographie en couleurs comprenant au moins une couche d'émulsion à l'halogénure d'argent sensible au rouge, au moins une couche d'émulsion à l'halogénure d'argent sensible au vert et au moins une couche d'émulsion à l'halogénure d'argent sensible au bleu, qui contiennent, dans l'ordre indiqué, au moins un copulant pour le bleu-vert, au moins un copulant pour le magenta et au moins un copulant pour le jaune.

**4.** Matériau d'enregistrement photographique selon l'une quelconque des revendications 1 à 3, caractérisé en ce qu'il s'agit d'un matériau inversible en couleurs.

**5.** Matériau d'enregistrement photographique selon l'une quelconque des revendications 1 à 4, caractérisé en ce qu'au moins une des couches d'émulsion à l'halogénure d'argent utilisées possède une teneur en iodure $\geqq$ 4 moles% et un diamètre moyen de particule $\leqq$ 0,4 µm.